# EUROPÄISCHE PATENTANMELDUNG

(11) **EP 0 534 304 A1**
(43) Veröffentlichungstag der Anmeldung: **31.03.1993**
(21) Anmeldenummer: 92115834.1
(22) Anmeldetag: 16.09.1992
(51) Int. Cl.: A61K 31/785, C08G 73/04

(54) **Cycloalkylierte Polyethyleniminderivate und ihre Verwendung als Hypolipidämika**

(30) Priorität: 21.09.1991 DE 4131507
(71) Anmelder: HOECHST AKTIENGESELLSCHAFT, D-65926 Frankfurt am Main (DE)
(72) Erfinder: Müllner, Stefan, Dr., W-6203 Hochheim (DE); Glombik, Heiner, Dr., W-6238 Hofheim am Taunus (DE)

(57) **Zusammenfassung**

Es werden unvernetzte, alkylierte Polyethylenimine beschrieben, die aufgrund ihrer gallensäurebindenden Eigenschaften als Hypolipidämika und Zusätze zu Lebensmitteln und Fruchtsäften verwendet werden können.

## Beschreibung

Die Erfindung betrifft alkylierte Polyethyleniminderivate, ein Verfahren zu ihrer Herstellung, pharmazeutische Zubereitungen auf Basis dieser Verbindungen sowie ihre Verwendung als Arzneimittel, insbesondere zur Senkung erhöhter Lipidspiegel.

Unlösliche, basische, vernetzte Polymere werden seit geraumer Zeit zur Bindung von Gallensäure verwendet und aufgrund dieser Eigenschaften therapeutisch genutzt. As Therapieobjekt werden die chologene Diarrhoe (z.B. nach Ileumresektion) und erhöhter Cholesterin-Blutspiegel kausal behandelt. Im letzteren Fall handelt es sich um den Eingriff in den enterohepatischen Kreislauf, wobei anstelle des aus dem Kreislauf genommenen Gallensäureanteils die entsprechende Neusynthese aus Cholesterin in der Leber provoziert wird. Zur Deckung des Cholesterinbedarfs in der Leber wird auf das zirkulierende LDL (Low Density Lipoprotein)-Cholesterin zurückgegriffen, wobei die hepatischen LDL-Rezeptoren in vermehrter Anzahl zur Wirkung kommen. Die so erfolgte Beschleunigung des LDL-Katabolismus wirkt sich durch die Herabsetzung des atherogenen Cholesterinanteils im Blut aus.

Die als Arzneimittel in der Verwendung befindlichen Ionenaustauscher besitzen als aktive Gruppen entweder quartäre Ammoniumgruppen (wie Colestyramin) oder sekundäre bzw. tertiäre Aminogruppen (wie Colestipol). Die Tagesdosis an Colestyramin betragt zweckmäßigerweise 12 - 24 g, als Tageshöchstdosis werden 32 g empfohlen. 15 - 30 g ist die empfohlene tägliche Colestipol-Dosis. Geschmack, Geruch und hohe Dosierung erschweren die Patienten-Compliance. Die Nebenwirkungen gehen auf mangelnde Selektivität (z.B. Avitaminosen) zurück, die auch bei der Dosierung simultan gegebener Medikamente berücksichtigt werden müssen, aber auch auf Gallensäureverarmung, die verschiedene gastrointestinale Störungen (Obstipation, Steatorrhoe) unterschiedlichen Grades hervorrufen. Für beide Präparate wurde eine therapeutische Bedeutung durch Kombination mit anderen hypolipidämisch wirkenden Pharmaka wie Fibrate, HMG-CoA-Reduktase-Inhibitoren, Probucol (vgl. z.B. M.N. CAYEN, Pharmac. Ther. 29, 187 (1985) und 8th International Symposium on Atherosclerosis, Rome, Oct. 9-13, 1988, Abstracts S. 544, 608, 710)) beschrieben, wobei die erzielten Effekte auch die Therapie von schweren Hyperlipidämien ermöglichen. Deshalb erscheint es bedeutungsvoll, bei dem gegebenen Wirkprinzip, geeignete Stoffe ohne die Nachteile der gegenwärtig verwendeten Präparate zu finden.

Folgende Merkmale der genannten Präparate und insbesondere von Colestipol sind als verbesserungswürdig anzusehen:
1. Die hohen Tagesdosen, die zurückzuführen sind auf eine relativ geringe Bindungsrate bei neutralem pH in isotonem Medium und der (teilweisen) Wiederfreisetzung der adsorbierten Gallensäure.
2. Die qualitative Verschiebung der Gallensäurezusammensetzung der Galle mit abnehmender Tendenz für Chenodesoxycholsäure und die damit verbundene zunehmende Gefahr für Cholelithiasis.
3. Das Fehlen einer dämpfenden Wirkung auf den Cholesterinstoffwechsel der Darmbakterien.
4. Die zu hohe Bindungsrate von Vitaminen und Pharmaka macht einen Substitutionsbedarf an diesen Stoffen und Blutspiegelkontrollen eventuell notwendig.
5. Eine weitere Verbesserung kann in der Darreichungsform erzielt werden.

Die Behebung der aufgelisteten Mängel gelingt überraschenderweise durch die Anwendung hochmolekularer alkylierter Polyethylenimine. Die nicht resorbierbaren Makromoleküle entfalten ihre Wirkung sowohl in löslicher Form als auch pH-abhängig in unlöslicher Form, entsprechend der unvernetzten Struktur, als auch in unlöslichem Zustand als vernetzte Polymere.

Vernetzte Polyethylenimine werden in der US-Patentschrift 3 332 841 beschrieben. Die Vernetzung erfolgt u.a. über Alkylengruppen mit 2 bis 8 Kohlenstoffatomen, das Molekulargewicht der Ausgangspolymeren liegt zwischen 800 und 100 000. Zur Behandlung von vorübergehender Hyperacidität des Magens werden 0,25 bis 5 g pro Dosierungseinheit verabreicht. Es wird weder die Bindung von Gallensäure noch eine damit verbundene lipidsenkende Aktivität der vernetzten Polyethylenimine beschrieben, denn ohne Alkylierung besitzen die Polyethylenimine gegenüber den Gallensäuren je nach Typ keine oder nur unbedeutende Bindungskapazität. Durch die große potentielle Ladungsdichte kann durch die Alkylierung für die ausreichende Bindungskapazität und durch die Wahl der Substituenten von entsprechend hydrophil/hydrophobem Charakter für die Affinität und Bindungsspezifität gesorgt werden.

In der DE-A-39 01 527 (entsprechend US-Patentanmeldung Nr. 07/466,923 bzw. Nr. 07/762,177 und entsprechend EP-A-0 379 161) werden unvernetzte und vernetzte alkylierte Polyethyleniminederivate beschrieben, bei denen das Grundpolymer Molekulargewichte von 10 000 bis 10 000 000 aufweist und die Alkylierungsmittel der allgemeinen Formel R-X entsprechen, wobei X ein Halogen, Pseudohalogen oder halogen-ähnliche Verbindung ist, R ein geradkettiger oder verzweigter C₁-C₃₀-Alkylrest ist, und im Falle der vernetzten alkylierten Polyethylenimine das Vernetzungsmittel ein alpha, omega-Dihalogenalkan mit 2-10 Kohlenstoffatomen oder ein höher funktionelles Halogenalkan mit 2-10 Kohlenstoffatomen ist.

Aufgabe der Erfindung war es nun Gallensäureadsorber auf Polyethyleniminbasis bereitzustellen, die in verbesserter Weise in der Lage sind, erhöhte Cholesterinspiegel zu senken und eine erhöhte und selektivere Bindung mit Gallensäure eingehen.

Überraschenderweise wurden nun gefunden, daß unvernetzte cycloalkylierte Polyethylenimine eines Molekulargewichtes von 10 000 bis 10 000 000, diese Aufgabe in hervorragender Weise erfüllen.

Gegenstand der Erfindung sind daher unvernetzte cycloalkylierte Polyethylenimine, herstellbar aus einem Ausgangspolyethylenimin eines Molekulargewichts von 10 000 bis 10 000 000 und einem Cycloalkylierungsmittel der Formel I

R-X (I)

worin
- R: ein Cycloalkylrest mit 5-30 Kohlenstoffatomen der monocyclisch, bicyclisch, tricyclisch, polycyclisch und/oder verbrückt sein kann und
- X: Chlor, Brom, Jod, CH₃-SO₂-O- oder 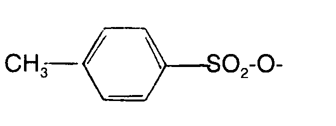
bedeutet.

Insbesondere bevorzugt sind unvernetzte Polyethylenimine, in denen R Cyclopentyl, Cyclohexyl, Cycloheptyl, Cyclooctyl, Cyclononyl, Cyclodecyl, bicyclische Systeme wie Decalyl, Hydrindanyl, verbrückte Systeme wie Norbornyl, oder polycyclische Systeme wie z.B. Cyclopentanoperhydrophenanthren und davon abgeleitete Ringsysteme oder Derivate bedeutet.

Gegenstand der Erfindung ist ebenso ein Verfahren zur Herstellung von unvernetzten Polyethyleniminen, dadurch gekennzeichnet, daß man nach in der Polymerchemie üblichen Methoden ein Polyethylenimin mit einem Molekulargewicht zwischen 10 000 und 10 000 000 mit einem Alkylierungsmittel der Formel R-X, worin X und R die angegebenen Bedeutungen haben, alkyliert.

Vorzugsweise werden Polyethylenimine mit einem Molekulargewicht über 100 000 eingesetzt.

In den Alkylierungsmitteln R-X ist X vorzugsweise Chlor oder Brom.

Das Verhältnis des eingesetzten Alkylierungsmittels zu den Aminogruppen des Polyethylenimins beträgt 0.2 : 1 bis 5 : 1, vorzugsweise 0.5 : 1 bis 2 : 1.

Durch die Reaktion mit Alkylierungsmittel wird ein Teil der sekundären Aminogruppen der Kette in tertiäre und quartäre Strukturen überführt. Die Bildung von tertiären Aminogruppen wird bevorzugt.

Gegenstand der Erfindung ist ebenso ein pharmazeutisches Präparat, welches eine oder mehrere der erfindungsgemäßen Polyethylenimine oder deren physiologisch verträgliche Salze einer Säure enthält.

Weiterhin betrifft die Erfindung die Verwendung der erfindungsgemäßen Verbindungen zur Herstellung eines Arzneimittels. Die Verbindungen können gelöst oder suspendiert in pharmakologisch unbedenklichen organischen Lösungsmitteln, wie ein- oder mehrwertigen Alkoholen, z.B. Ethanol oder Glycerin, in Triacetin, Ölen, z.B. Sonnenblumenöl, Lebertran, Ethern, z.B. Diethylenglykoldimethylether, oder auch Polyethern, z.B. Polyethylenglykol, oder auch in Gegenwart anderer pharmakologisch unbedenklicher Polymerträger, wie z.B. Polyvinylpyrrolidon, oder anderen pharmazeutisch annehmbaren Zusatzstoffen wie Lactose, Stärke, Gelatine, Cyclodextrin, Talkum oder Polysacchariden angewendet werden. In fester Form anfallende Wirkstoffe werden zunächst z.B. durch Feinmahlung auf eine gewünschte Partikelgröße gebracht. Ferner können die erfindungsgemäßen Verbindungen in Kombination mit anderen Arzneistoffen gegeben werden, z.B. mit HMG-CoA-Reduktasehemmern, Vitaminen, Geriatrika und Antidiabetika.

Die erfindungsgemäßen Verbindungen werden in verschiedenen Dosierungsformen verabreicht, vorzugsweise oral in Form von Tabletten, emulgierbare Zubereitungen, Kapseln oder Flüssigkeiten, wie z.B. auch Lebensmitteln oder Fruchtsäften.

Die erfindungsgemäßen Verbindungen können verwendet werden als Arzneimittel, insbesondere zur Hemmung der Gallensäurerückresorption im Darm und als Hypolipidämikum und bei allen Erkrankungen, bei denen erhöhte Gallensäure-Konzentrationen im Darm oder Serum auftreten.

Ebenso können die erfindungsgemäßen Verbindungen verwendet werden als Zusatz in Lebensmitteln, insbesondere in Fruchtsäften, Brot usw. oder zusammen mit diesen eingenommen werden.

Zu den Gallensäuren zählen natürliche Gallensäuren, Derivate der natürlichen Gallensäuren und deren Alkali- bzw. Erdalkalisalze. Insbesondere zählen hierzu: Glycocholat, Taurocholat, Cholat, Cholsäure, Desoxy-, Chenodesoxy-, Ursodesoxy- und Lithocholsäure sowie deren Konjugate mit Glycin oder Taurin. Zusätzlich kann auch Cholesterin adsorbiert werden.

Unter Serum wird insbesondere Human- oder Animal-Serum verstanden.

Die erfindungsgemäßen alkylierten Polyethylenimine adsorbieren körpereigene Säuren, insbesondere Gallensäuren. Aufgrund dieser Eigenschaften sind sie in der Lage erhöhte Cholesterinspiegel zu senken. Die erfindungsgemäßen alkylierten Polyethylenimine besitzen gegenüber Colestipol wesentlich günstigere gallensäureadsorbierende Eigenschaften wie aus den nachstehenden beschriebenen Versuchen erkennbar ist.

Hinsichtlich der Verträglichkeit und der Selektivität der Gallensäurebindung, sowie der simultanen Cholesterinbindung, zeigt sich sowohl in-vitro- als auch in-vivo-Versuchen in Wistar-Ratten eine deutliche Überlegenheit (soft-gel-Charakteristik).

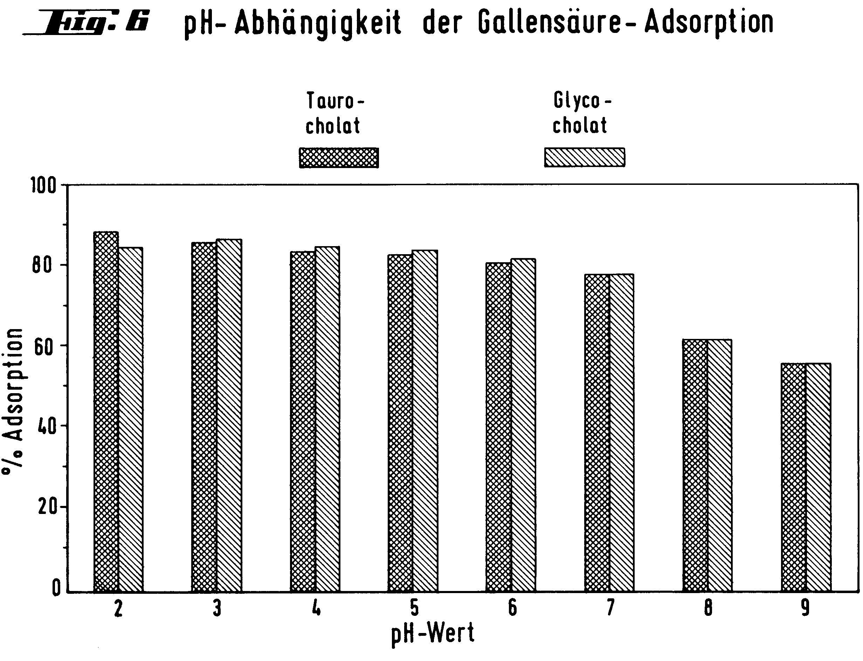

Figur 6 zeigt die pH-Abhängigkeit der Gallensäurebindung, welche über den physiologischen pH-Bereich nahezu Konstant bleibt und erst oberhalb pH 8 nachläßt.

Diese Eigenschaften des unvernetzten alkylierten Polymers gewährleisten sowohl im Magen (Gallenflüssigkeit-Reflux bei Lebererkrankungen) als auch in leicht basischem Medium des Dünndarms, sowie in der leicht aciden Umgebung im Mastdarm eine gute bis optimale Gallensäurebindung.

### Beispiel

4,3 g (0,1 mol) Polyethylenimin (in 50 % wäßriger Lösung) werden mit 100 ml Wasser verdünnt und mit 23,7 g (0,2 mol) Chlorcyclohexan 24 h unter starkem Rühren zum Rückfluß erhitzt. Nach kurzer Abkühlung gibt man 100 ml 2N NaOH hinzu und erhitzt erneut 24 h zum Rückfluß.

Die organische Phase wird abgetrennt. Die wäßrige Phase wird mit Dichlormethan gewaschen und am rotationsverdampfer und danach im Ölpumpenvakuum eingeengt. Der Rückstand wird aus wäßriger Lösung dialysiert.

Nach Entfernen des Wassers (Rotationsverdampfer, Ölpumpenvakuum Gefriertrocknung) erhält man das mit Cyclohexan substituierte Polyethylenimin als schwach gelbliches Pulver (Charakterisierung durch Elementaranalyse).

### 1. In vitro Versuche

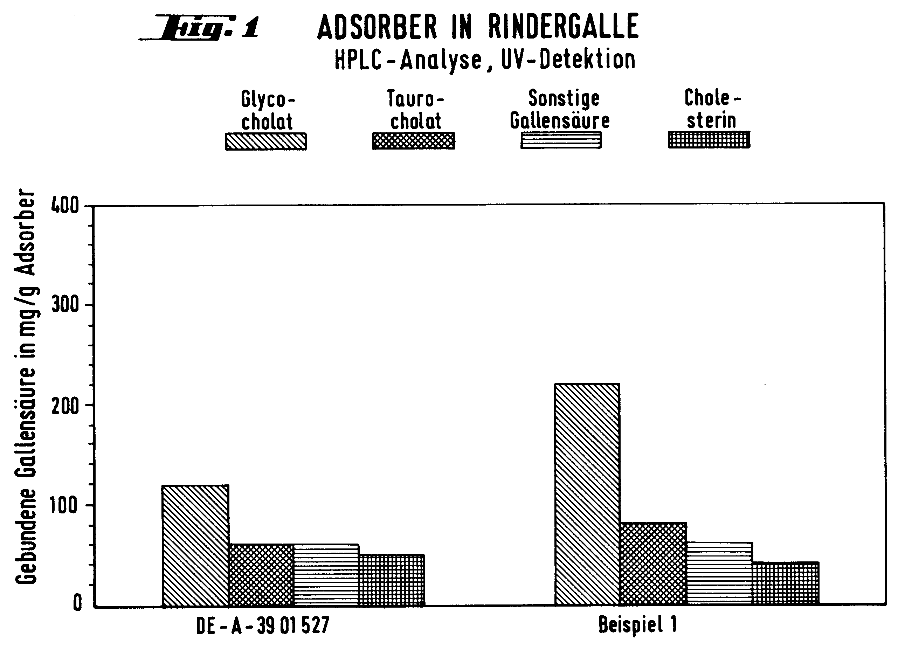

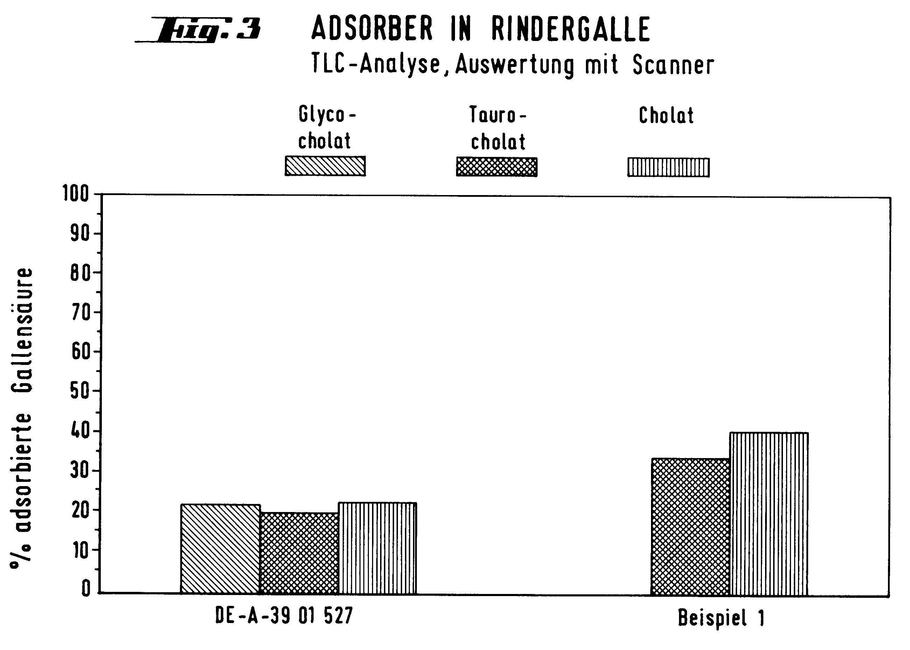

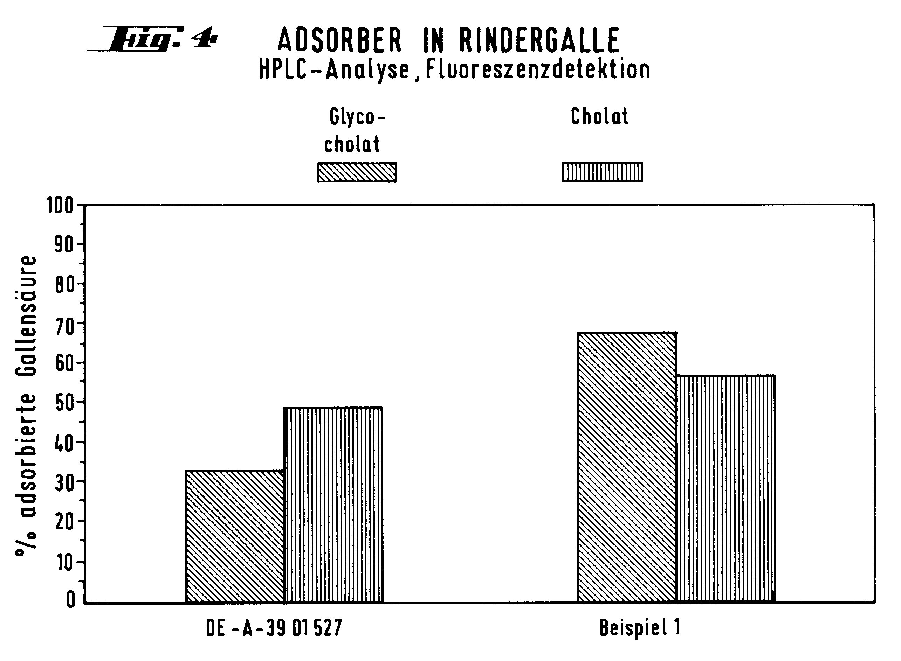

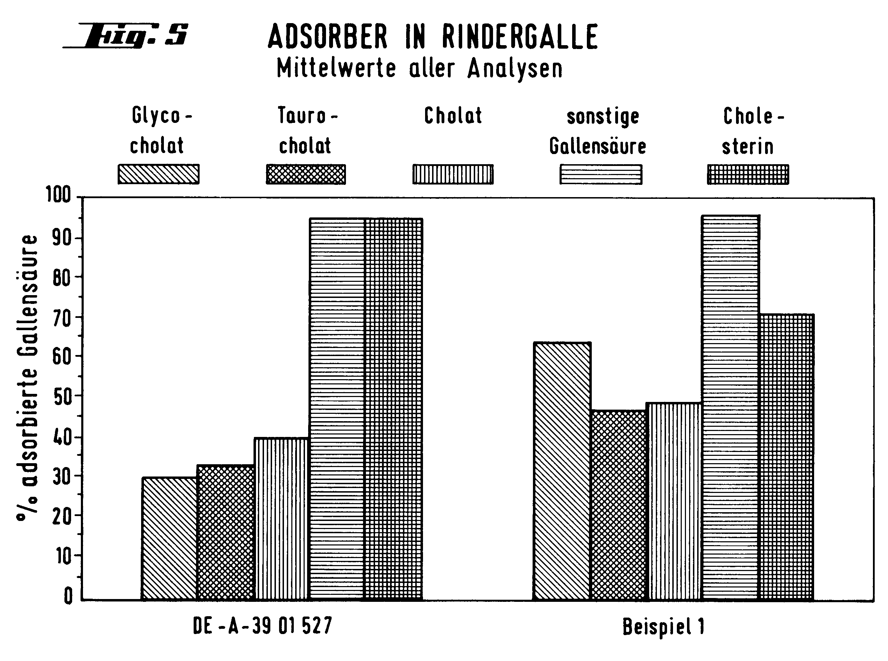

Die In-vitro-Versuche zur Überprüfung der Bindungseigenschaften von den erfindungsgemäßen Verbindungen wurden in einem eigens dafür entwickelten Rindergalle-Assay überprüft. Es wurde nämlich gefunden, daß die Bindungsaffinität und Kapazität von Gallensäure-Adsorbentien durch Ioneneinflüsse und pH-Wert (Figur 6) stark differieren kann und auch abhängig vom jeweiligen Inkubationsmedium ist. Von der allgemeinen Zusammensetzung ist Rindergalle sehr ähnlich der Humangalle und in reproduzierbarer Qualität sowie in ausreichender Quantität erhältlich. Wegen der Nähe zu den im Duodenum herrschenden physiologischen Bedingungen und der zu erwartenden besseren Übertragbarkeit der In-vitro-Daten auf die Tierversuchsdaten bzw. klinischen Daten, wurde daher anstatt des sonst üblichen Bindungstests von käuflichen, reinen Gallensäure in reinem physiologischen Puffer ein Bindungsassay auf Rindergalle-Basis entwickelt. Die Figuren 1 bis 5 zeigen die Auswertung des Adsorbertests in Rindergalle mit entsprechender UV-HPLC, Fluoreszenz-HPLC und TLC-Auswertung. Verglichen werden Adsorber aus der DE-A-3 901 527 (Beispiel 2) mit der erfindungsgemäßen Substanz aus Beispiel 1.

### Legende zu den Figuren 1 bis 6

### Figur 1

Das Diagramm zeigt die HPLC-Analyse (UV-Detektion) des Bindungsverhaltens der Verbindung gemäß Beispiel 1 der Erfindung und der Verbindung gemäß Beispiel 2 aus DE-A-3 901 527 (Vergleichsverbindung).
Der Überstand der verdünnten Rindergalle (1:10 mit 50 mM NaHCO₃, pH 6,5) wurde nach 24 h Inkubation (bei 37°C) von 10 mg/ml Gallensäure-Sequestrants analysiert.
Die Ergebnisse sind in Form von gebundener Gallensäure/g Adsorber dargestellt.

### Figur 2

Das Diagramm zeigt die HPLC-Analyse (UV-Detektion) des Bindungsverhaltens der Verbindung nach Beispiel 1 der Erfindung, der Verbindung gemäß Beispiel 2 aus DE-A-3 901 527 und von kommerziell erhältlichem Colestipol.
Der Überstand der verdünnten Rindergalle (1:10 mit 50 mM NaHCO₃, pH 6,5) wurde nach 24 h Inkubation (bei 37°C) von 10 mg/ml Gallensäure-Sequestrants analysiert.
Die Ergebnisse sind in % gebundene Gallensäure (Taurocholat und Glykocholat) dargestellt.

### Figur 3

Das Diagramm zeigt die TLC (Dünnschicht-Chromatographie) Analyse (Fluoreszenz-Scan) des Bindungsverhaltens der Verbindung nach Beispiel 1 der Erfindung und der Verbindung gemäß Beispiel 2 aus DE-A-3 901 527 (Vergleichsverbindung).
Der Überstand der verdünnten Rindergalle (1:10 mit 50 mM NaHCO₃, pH 6,5) wurde nach 24 h Inkubation (bei 37°C) von 10 mg/ml Gallensäure-Sequestrants analysiert.
Die Ergebnisse sind als % gebundene Gallensäure (Cholat, Taurocholat und Glykocholat) dargestellt.

### Figur 4

Das Diagramm zeigt die HPLC-Analyse (Fluoreszenzdetektion) des Bindungsverhaltens der Verbindung nach Beispiel 1 der Erfindung und der Verbindung gemaß Beispiel 2 aus DE-A-3 901 527 (Vergleichsverbindung).
Der Überstand der verdünnten Rindergalle (1:10 mit 50 mM NaHCO₃, pH 6,5) wurde nach 24 h Inkubation (bei 37°C) von 10 mg/ml Gallensäure-Sequestrants analysiert.
Die Ergebnisse sind als % gebundene Gallensäure (Cholat und Glykocholat) dargestellt.

### Figur 5

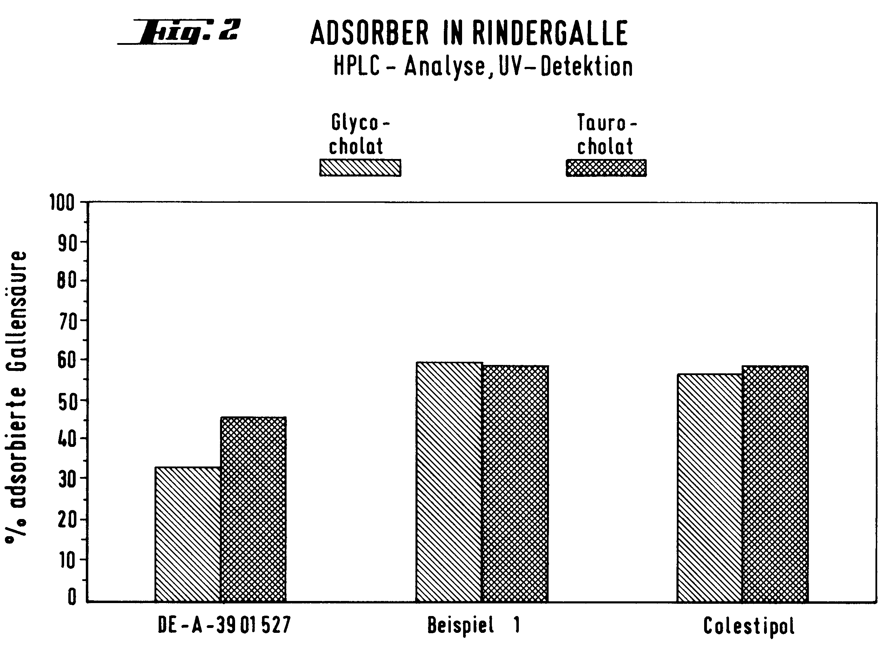

Das Diagramm zeigt die Mittelwerte aller Analysen (Figuren 2, 3 und 4). Das Bindungsverhalten der Verbindung nach Beispiel 1 der Erfindung ist demjenigen der Verbindung gemäß Beispiel 2 aus DE-A-3 901 527 gegenübergestellt.

### Figur 6

Das Diagramm zeigt die pH-Abhängigkeit der Gallensäure-Bindung der Verbindung nach Beispiel 1 der Erfindung.
Der Überstand der verdünnten Rindergalle (1:10 mit 50 mM NaHCO₃, pH 6,5) wurde nach 24 h Inkubation (bei 37°C) von 10 mg/ml Gallensäure-Sequestrants analysiert.
Die Ergebnisse sind als % gebundene Gallensäure (Taurocholat und Glykocholat) dargestellt und wurden mit HPLC (UV-Detektion) ermittelt.

### Testaufbau mit Rindergalle

- Rindergalle (vom Schlachthof, mit Zusatz von 0,02 % Natriumazid, aufbewahrt im Kühlschrank bei + 4°C) wird 1:10 mit Natriumbicarbonat-Puffer 50 mM, pH 6,5 verdünnt. Dadurch soll eine weitgehende Anpassung an die im Dünndarm herrschenden Verhältnisse erreicht werden (Information von Prof. A.F. Hoffman, University of California, San Diego, USA: 90 % der durch Gallensäureadsorber gebundenen Substanzen sind keine Gallensäuren, sondern vorwiegend Hydrogencarbonat.). Der pH-Wert von 6,5 entspricht dem der unverdünnten Rindergalle.
- Je 100 mg der zu testenden Adsorber werden in Erlenmeyerkolben mit Schliff eingewogen.
- Nach Zugabe von 10 ml der Galle-Puffer-Mischung werden die Ansätze verschlossen und in einem Schüttelwasserbad 24 h bei 37°C inkubiert.
- Zu jedem Test gehören zwei Kontrollansätze ohne Präparatezugabe und zwei Standardansätze mit Colestipol (reiner Wirkstoff). Pro Präparat wird eine Doppelbestimmung durchgeführt.
- Nach der Inkubation werden die Proben in Zentrifugenröhrchen umgefüllt und 10 min bei 6000 rpm abzentrifugiert.
- Der Überstand wird abgenommen und wie folgt analysiert:

### a.) Enzymatische Bestimmung der Gesamtgallensäure

- In Eppendorfgefäße werden je 900 µl des folgenden Gemisches gegeben:
   6 ml Tetra-Natrium-Diphosphat-Puffer 0,1 M, pH 8,9
   2 ml NAD-Lösung (4 mg/ml Wasser)
   20 ml ®Millipore-Wasser
- Dazu werden 30 µl der Probe und 30 µl Enzymlösung pipettiert
- Enzymlösung: 3-alpha-Hydroxysteroiddehydrogenase 0,5 units/ml
- Die Ansätze werden gemischt und 2 h bei Raumtemperatur inkubiert.
- Anschließend Umfüllen in 1 ml-Einmalküvetten und Messung im Photometer bei 340 nm.
- Für Galleproben nur bedingt geeignet, da die grüne Farbe stört.

### b.) TLC mit Scannerauswertung und Fotografie (Einzelgallensäuren und Cholesterin)

- TLC-Platten:: Kieselgel 60-DC Fertigplatten, 20 x 20 cm, Schichtdicke 0,5 mm, mit Konzentrierungszone von Merck, Art. Nr. 11845
- Laufmittel:: Chloroform : Methanol : Eisessig 85:20:8
- Färbereagenz:: Mangan-II-Chlorid / Schwefelsäure
- Färbezeit:: ca. 20 min bei 120°C im Trockenschrank
- Probenvolumen:: 5 µl n
- Auswertung:: a. "optisch" unter der UV-Lampe
b. mit Scanner der Fa. Camag und Auswertesoftware
c. Fotografie

### c.) HPLC mit UV-Detektion (konjugierte Gallensäuren)

Geräte: HPLC-Anlage der Fa. Kontron, bestehend aus drei Pumpen und Mischkammer, Autosampler, UV-Detektor und Auswerteeinheit mit Software MT2.
Mobile Phase: Laufmittel A: Ammoniumcarbamatpuffer 0,019 M, mit Phosphorsäure auf pH 4,0 eingestellt. Laufmittel B: Acetonitril
Säule: ®LiChrospher 100 RP-8, 25 mm, 5 µm von Merck
Vorsäule: LiChrospher 60 RP-select B, 4 mm, 5 µm von Merck
Flußrate:
Gradient:

| | |
|---|---|
| 0,00 min | 0,8 ml/min |
| 20,00 min | 0,8 ml/min |
| 23,00 min | 1,3 ml/min |
| 51,00 min | 1,3 ml/min |

Detektion: 200 nm (für Präparate zusätzlich bei 254 nm)
Gradient:

| | |
|---|---|
| 0,00 min | 32 % B |
| 8,00 min | 35 % B |
| 17,00 min | 38 % B |
| 20,00 min | 40 % B |
| 24,00 min | 40 % B |
| 30,00 min | 50 % B |
| 45,00 min | 60 % B |

### d.) Derivatisierung, HPLC mit Fluoreszenzdetektion (unkonjugierte und glycinkonjugierte Gallensäuren)

Geräte: HPLC-Anlage der Fa. Kontron, bestehend aus drei Pumpen und Mischkammer, Autosampler, UV-Detektor und Auswerteeinheit mit Software MT2. Fluoreszenzdetektor von Merck-Hitachi. Da die Proben licht- und temperaturempfindlich sind, wird der Autosampler auf ca. 5°C gekühlt.
Mobile-Phase: Laufmittel A: Millipore-Wasser (eigene Anlage) Laufmittel B: Acetonitril/Methanol 60:30
Säule: LiChrospher 100 RP-18, 25 mm, 5 µm von Merck
Vorsäule: LiChrospher 60 RP-select B, 4 mm, 5 µm von Merck
Flußrate: 1,3 ml/min
Detektion: Excitation: 340 nm
Emission: 410 nm
Gradient:

| | |
|---|---|
| 0,00 min | 66 % B |
| 7,50 min | 66 % B |
| 8,00 min | 76 % B |
| 12,50 min | 76 % B |
| 13,00 min | 83 % B |
| 25,00 min | 83 % B |
| 25,50 min | 91 % B |
| 40,00 min | 91 % B |

### e.) HPLC (Cholesterin)

- Geräte:: HPLC-Anlage der Fa. Kontron, bestehend aus drei Pumpen und Mischkammer, Autosampler, UV-Detektor und Auswerteeinheit mit Software MT2.
- Mobile Phase:: Acetonitril : Isopropanol 50:50
- Säule:: LiChrospher 100 RP-18, 25 mm, 5 µm von Merck
- Vorsäule:: LiChrospher 60 RP-select B, 4 mm, 5 µm von Merck
- Flußrate:: 1,0 ml/min
- Detektion:: 207 nm

### f.) "optisch" (Gallenfarbstoff)

### 1.2 Reversibilitätsprüfung

Das bei einem Ansatz wie oben beschrieben aus dem Gleichgewicht entfernte Adsorbat wurde mit frischem gallensäurefreien Medium, z.B. Wasser oder Phosphatpuffer, nachinkubiert und wie oben bestimmt.

## Patentansprüche

1. Unvernetzte alkylierte Polyethylenimine herstellbar aus einem Ausgangspolyethylenimin mit einem Molekulargewicht von 10 000 bis 10 000 000 und einem Alkylierungsmittel, dadurch gekennzeichnet, daß das Alkylierungsmittel die allgemeine Formel I besitzt
R - X (I)
worin
R ein Cycloalkylrest mit 5-30 Kohlenstoffatomen bedeutet und
X Chlor, Brom, Jod, CH₃-SO₂-O- oder 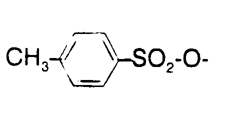
bedeutet.

2. Polyethylenimine nach Anspruch 1, dadurch gekennzeichnet, daß der Cycloalkylrest mono-, bi-, tricyclisch oder polycyclisch ist und/oder verbrückt ist.

3. Polyethylenimine nach den Ansprüchen 1 oder 2, dadurch gekennzeichnet, daß
R Cyclopentyl, Cyclohexyl, Cycloheptyl, Cyclooctyl, Cyclononyl, Cyclodecyl,
Decalyl, Hydrindanyl, Norbornyl oder Cyclopentanoperhydrophenanthren und deren Derivate ist.

4. Polyethylenimine nach den Ansprüchen 1 bis 3, dadurch gekennzeichnet, daß das Ausgangspolyethylenimin ein Molekulargewicht von größer als 100 000 aufweist.

5. Verfahren zur Herstellung von unvernetzten alkylierten Polyethyleniminderivaten, dadurch gekennzeichnet, daß man nach in der Polymerchemie üblichen Methoden ein Ausgangspolyethylenimin mit einem Molekulargewicht von 10 000 bis 10 000 000 mit einem Alkylierungsmittel der Formel I gemäß Anspruch 1 alkyliert.

6. Verfahren nach Anspruch 5, dadurch gekennzeichnet, daß eine oder mehrere der folgenden Maßnahmen eingehalten werden:
a) Polyethylenimine mit einem Molekulargewichtsbereich über 100 000 eingesetzt werden,
b) in den Alkylierungsmitteln R-X, X Chlor oder Brom ist,
c) in den Alkylierungsmitteln R ein cyclischer Alkylrest ist,
d) das Verhältnis des eingesetzten Alkylierungsmittels zu den Aminogruppen des Polyethylenimins 0,5 : 1 bis 2 : 1 beträgt.

7. Pharmazeutisches Präparat, dadurch gekennzeichnet, daß es eine Verbindung gemäß den Ansprüchen 1 bis 4 oder deren physiologisch verträgliches Salz mit einer Säure enthält.

8. Verfahren zur Herstellung eines pharmazeutischen Präparates, dadurch gekennzeichnet, daß man eine Verbindung gemäß den Ansprüchen 1 bis 4 in eine geeignete Darreichungsform überführt.

9. Verwendung einer Verbindung gemäß den Ansprüchen 1 bis 4 als Hypolipidämikum.

10. Verwendung von Verbindungen gemäß den Ansprüchen 1 bis 4 als Zusatz in Lebensmitteln und Fruchtsäften.

## Patentansprüche (Patentansprüche für folgende(n) Vertragsstaat(en): ES, GR)

1. Verfahren zur Herstellung von unvernetzten, alkylierten Polyethyleniminen, dadurch gekennzeichnet, daß man ein Ausgangspolyethylenimin mit einem Molekulargewicht von 10 000 bis 10 000 000 mit einem Alkylierungsmittel, der Formel I
R - X (I)
worin
R ein Cycloalkylrest mit 5-30 Kohlenstoffatomen bedeutet und
X Chlor, Brom, Jod, CH₂-SO₂-O- oder 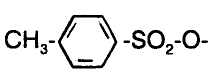
bedeutet, nach in der Polymerchemie üblichen Methoden alkyliert.

2. Verfahren nach Anspruch 1, dadurch gekennzeichnet, daß man eine Verbindung I, worin der Cycloalkylrest mono-, bi-, tricyclisch oder polycyclisch ist und/oder verbrückt ist, verwendet.

3. Verfahren nach den Ansprüchen 1 oder 2, dadurch gekennzeichnet, daß man ein Alkylierungsmittel der Formel I, worin
R Cyclopentyl, Cyclohexyl, Cycloheptyl, Cyclooctyl, Cyclononyl, Cyclodecanyl, Decalyl, Hydrindanoyl, Norbornyl oder Cyclopentanoperhydrophenanthren und deren Derivate ist, verwendet.

4. Verfahren nach Anspruch 1, dadurch gekennzeichnet, daß eine oder mehrere der folgenden Maßnahmen eingehalten werden:
a) Polyethylenimine mit einem Molekulargewichtsbereich über 100 000 eingesetzt werden,
b) in den Alkylierungsmitteln R-X, X Chlor oder Brom ist,
c) in den Alkylierungsmitteln R ein cyclischer Alkylrest ist,
d) das Verhältnis des eingesetzten Alkylierungsmittels zu den Aminogruppen des Polyethylenimins 0,5 : 1 bis 2: 1 beträgt.

5. Verfahren zur Herstellung eines pharmazeutischen Präparates, dadurch gekennzeichnet, daß man eine Verbindung erhältlich nach Verfahren der Ansprüchen 1 bis 4 in eine geeignete Darreichungsform überführt.
